# EUROPEAN PATENT SPECIFICATION

(11) **EP 1 093 767 B1**
(45) Date of publication and mention of the grant of the patent: **23.03.2005**
(21) Application number: 99308320.3
(22) Date of filing: 21.10.1999
(51) Int. Cl.: A61C 8/00, A61B 10/00

(54) **Combination of syringe and aspirator for bone regeneration material**
Kombination von Spritze und Sauger für Knochenregenerationsmaterial
Conbination d'une syringe et d'un aspirateur pour substance de régénération d'os.

(43) Date of publication of application: 25.04.2001
(73) Proprietor: Ashman, Arthur, Westport, CT 06880 (US)
(72) Inventor: Ashman, Arthur, Westport, CT 06880 (US)
(74) Representative: Marchant, James Ian

(56) References cited:
- WO-A-98/16268
- US-A- 4 366 822
- US-A- 4 911 641
- US-A- 5 269 785
- US-A- 5 330 357
- US-A- 5 824 087

## Description

### BACKGROUND OF THE INVENTION

The invention relates to a nozzle tip of special construction mounted on the barrel of a standard syringe for dispensing bone regeneration materials to a surgical site. The nozzle tip and syringe are used to aspirate marrow blood from a surgical site; then mixing the collected blood marrow with granular bone regeneration material stored in the barrel of the syringe to form a viscous fluid mixture therein; then manually removing the nozzle tip from the syringe barrel; and then dispensing the viscous fluid mixture to the surgical site by manual application of pressure on the plunger of the syringe. Bone regeneration materials are known in the art. For example, hard-tissue implant materials are known, such as the calcified microporous co-polymer material marketed under the trademarks Bioplant® HRT® *Synthetic Bone*^{*TM*} alloplast. The aforesaid bone regeneration material has been widely accepted in medicine, dentistry and veterinary medicine as a prostethic bone material to repair injured or diseased bone. The following co-invented U.S. patents describe the use of such bone generation materials: 4,199,864; 4,244,689; 4,535485; 4,536,158; 4,547,327; 4,547,390; and 4,728,570. The aforelisted co-invented U.S. patents are incorporated by reference herein. For many applications of said Bioplant®, HTR® material the application of this material in granular form has proven to have many advantages. For example, granular Bioplant®, HTR® material has proven particularly useful in a tooth extraction procedure. A simple injection of granular Bioplant®, HTR® material into the tooth socket, following immediately after extraction of the tooth, either significantly reduces or completely prevents the usual 40% to 60% percent bone loss that otherwise occurs within 2-3 years after tooth extraction, and eliminates much of the pain and inflammation of the tooth socket (post-extraction alveolar osterities). Granular Bioplant®, HTR®, material works best when it is thoroughly wetted with marrow blood before being applied to a surgical site.

U.S. Patent No. 4,911,641 discloses an open ended IMI syringe with a transparent barrel that contains a bone growth promoting composition. The open end portion of the syringe is placed adjacent to a bony defect (e.g. in the human jaw) and the composition expelled into the defect. Saliva and blood from the mouth and surrounding bone activate the composition and cause it to stick to itself and to the treated surface, allowing the bony defect to the overfilled.

U.S. Patent No. 4,366,822 discloses a bone marrow separation apparatus comprising a syringe of conventional construction, to the nozzle-shaped end of which is attached a filtration chamber and a needle. The syringe is used to induce withdrawal of sinusoidal blood and bone marrow particles from a bone of a mammal through said needle and filtration chamber, whereby said bone marrow particles are separated from the blood by a filter as the blood is drawn into the syringe.

### SUMMARY OF THE INVENTION

It is an object of the present invention to provide a simple improved syringe and nozzle tip construction for producing and then dispensing a viscous mixture of granular Bioplant®, HTR® material and marrow blood, obtained from a surgical site.

Aspirated marrow blood from a surgical site may be mixed with granular bone regeneration material inside the barrel of a syringe and, by the use of an improved nozzle tip construction according to the present invention, mounted on a standard syringe, excessive loss of marrow blood and/or granular bone regeneration material during the mixing operation is prevented.

Said mixing may be carried out in an aseptic manner, and subsequently the viscous mixture of marrow blood and granular bone regeneration material obtained by the mixing in the sterile syringe barrel may be applied to a surgical site in an aseptic manner.

Low density polyethylene has been found to be particularly advantageous for manufacturing the entire nozzle tip construction including the filter screen which is mounted inside the nozzle tip. The openings of the mesh screen must be smaller than the grain size of the granular bone regeneration material inside the syringe barrel. A mesh opening size of about 105 microns has been found to work best with the method of the invention because it can be used with several standard granular sizes of Bioplant®, HTR® materials.

Further details regarding the nozzle tip construction and its use in the method of forming a viscous mixture of granular Bioplant® HTR® polymer material and then applying it to a surgical site will be provided in the following description of preferred embodiments in conjunction with the accompanying drawings.

### BRIEF DESCRIPTION OF THE DRAWINGS

Figure 1 is a cross-sectional view of the nozzle tip of the invention;
Figure 2 is a cross-sectional view of a standard straight barrel syringe holding bone regeneration material, with a nozzle tip of the invention mounted thereon, which standard type of syringe is commonly used in applying bone regeneration materials to a surgical site;
Figure 3 is a cross-sectional view of the straight barrel syringe of Figure 2 with the nozzle tip of Figure 2 mounted thereon during the step of aspiration marrow blood from a tooth socket and mixing it with bone regeneration material in the syringe;
Figure 4 is a cross-sectional view of the straight barrel syringe of Figure 2 after the mixing step has been completed and the nozzle tip has been manually removed so that the viscous mass formed by the mixture of marrow blood and bone regeneration material is ready to be applied to a surgical site;
Figure 5 is a view in perspective showing the step of aspirating marrow blood from a tooth socket with the nozzle tip construction of the invention; and
Figure 6 is a view in perspective showing the step of applying the viscous mixture in the syringe barrel to the tooth socket.

### DESCRIPTION OF THE PREFERRED EMBODIMENTS

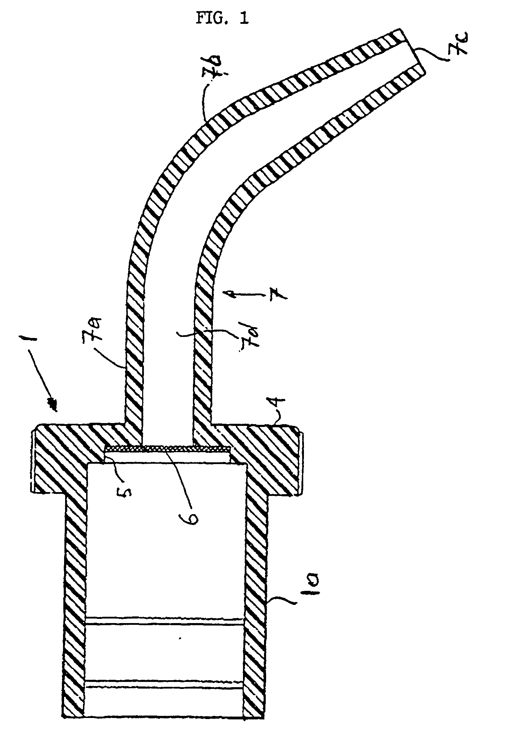

Although the principles of this invention are applicable to other surgical procedures than a tooth extraction, the invention will be fully understood from an explanation of its application to a preferred embodiment of a syringe and special nozzle tip construction as illustrated in Figures 1-6.

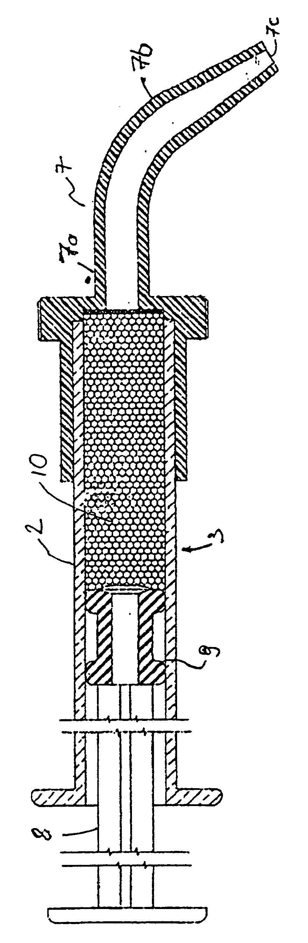

Shown in FIG. 1 is a cross-sectional view of the nozzle tip 1 of this invention. The nozzle tip 1 includes a sleeve portion 1a which has an internal diameter which corresponds to the external diameter of the barrel 2 of the syringe 3 illustrated in FIG.2. The nozzle tip 1 is therefore mounted on the syringe barrel 2 by means of a friction fit. The syringe 3 is of the type that is commonly used for dispensing granular bone regeneration material, such as Bioplant®, HTR® material. The nozzle tip has a flange 4 which has a recess 5. A screen 6 having a mesh size of about 105 microns is mounted inside the recess 5. The nozzle tip further has a neck portion 7 with a passage 7d extending therethrough. The neck portion 7 includes an axially straight portion 7a extending from the flange 4 and integral therewith, and a curved portion 7b through the opening 7c thereof the marrow blood can aspirated. The neck portion 7 is integral with the flange 4 and the entire nozzle tip construction including the neck portion 7 and screen 6 are preferably made by a known moulding operation of low density polyethylene.

FIG. 2 illustrates in cross-section a syringe 3 with the nozzle tip 1 mounted thereon. The barrel 2 of the syringe 3 is filled with a granular bone generation material 10, such as Bioplant®, HTR® material. This barrel is made of either glass or transparent plastic material. The syringe 3 further has the standard plunger 8 on the front end of which is mounted a piston 9. By applying manual pressure to the plunger 8 and the piston 9 can be reciprocally slidably axially moved inside the barrel 2 of the syringe 3. The entire assembly, as illustrated in Figure 2, is mounted inside a non-illustrated conventional blister pack, in which it is distributed to the dentist, surgeon or veterinary practitioner for application of the bone regeneration material to a surgical site. This entire assembly is intended for a single use only and the assembly and blister pack is intended to be discarded after this single use.

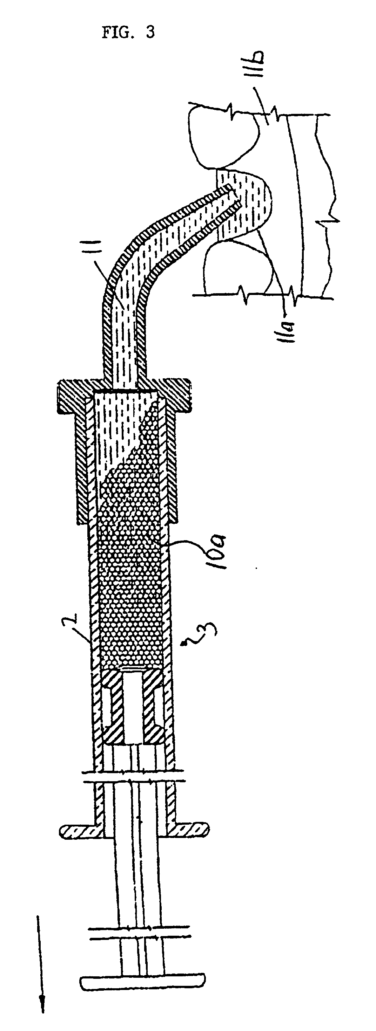

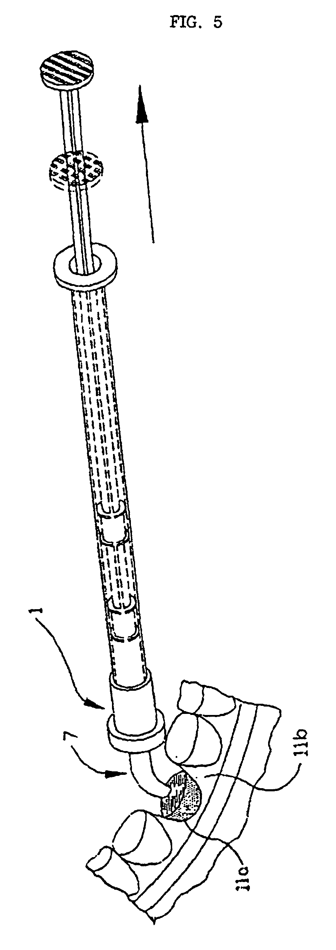

FIGS. 3 and 5 illustrate the aspirating step of the invention using the nozzle tip 1 and syringe 3 of the invention. The curved portion 7b of the nozzle tip 1 is inserted, by way of example, by the dentist into the tooth socket 11a of a jaw bone 11b of a patient immediately after a non-illustrated tooth has been extracted from the tooth socket 11a. Marrow blood 11 is then aspirated through the opening 7c of the neck portion 7 by manually retracting the plunger 8. The aspirated marrow blood 11 flows through neck portion 7 and the screen 6 into the barrel 2 of the syringe where it immediately begins to soak the bone regeneration material 10 with marrow blood 11. By visually examining the syringe 3 the dentist or surgeon determines when a sufficient marrow blood 11 has been aspirated from the tooth socket 11a and has mixed with the bone regeneration material 10. If an insufficient amount of marrow blood has been aspirated the afore-described steps are repeated. If excess marrow blood has been aspirated this excess marrow blood is expelled by slightly manually moving the plunger forward. While these steps are carried out the screen 6 prevents the clogging with granular bone regeneration material of the passage 7d in the straight neck portion 7a of the neck portion 7.

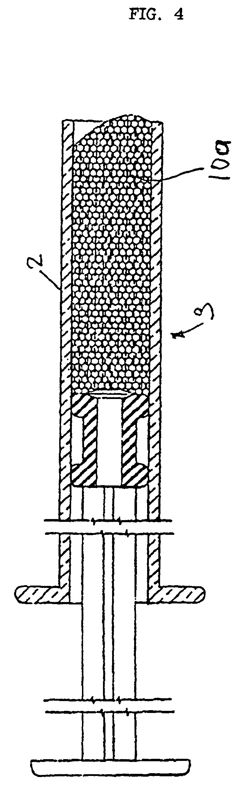

By visually examining the mixture of marrow blood and bone regeneration material inside the syringe barrel 2, the dentist can determine when the mixture 10a of bone regeneration material and marrow blood 11 contains a sufficient amount of marrow blood and thereby the mixture has become sufficiently viscous to be applied to a surgical site. The nozzle tip 1 is then manually slid off the syringe barrel 2 as is shown in FIG. 4.

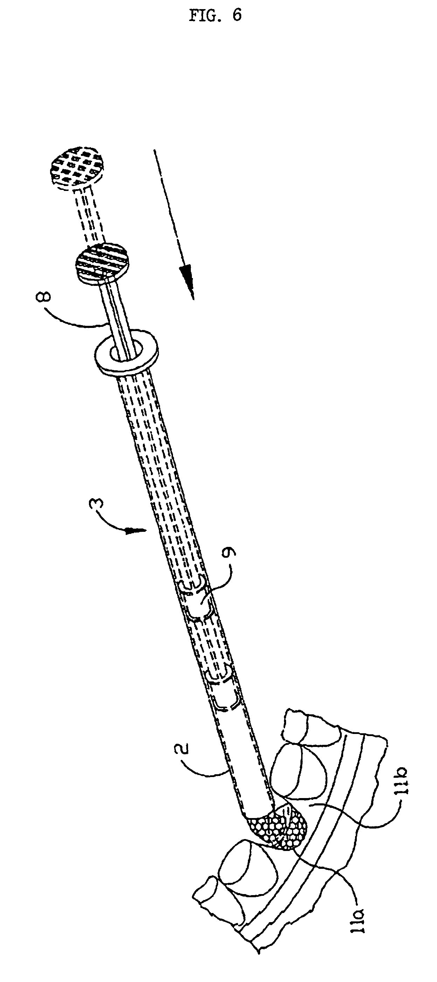

As is shown in FIG. 6 the viscous mixture 10a is then applied to a surgical site, such as a tooth socket 11a, by applying manual pressure to the plunger 8. Once this step has been completed the surgeon may apply sutures to the surgical site of the surgical condition of the patient warrants such a step.

Although the nozzle tip construction of the present invention has been described in terms of the presently preferred embodiments, it is to be understood that such disclosure is not to be interpreted as limiting. For example, it should be noted that the syringe assembly of the invention can be used in other surgical procedures that tooth extraction and can find application in surgery and veterinary medicine.

## Claims

1. A syringe and nozzle tip assembly for applying a viscous mixture of marrow blood and bone regeneration material to a surgical site, comprising in combination,
a) a syringe (3) having a syringe barrel (2) which has a front end and a rear end,
b) a piston slidably reciprocally movably mounted in said syringe barrel;
c) a plunger being connected at its front end to said piston and axially extending rearwardly from said syringe barrel through said rear end thereof; and
d) a nozzle tip (1) having a flange (4) and a sleeve portion (1a) which is coaxially mounted on the front end of said syringe barrel (2) by means of a friction fit; said nozzle tip (1) having a neck portion (7) with a passage extending therethrough for aspirating marrow blood from a surgical site and transporting it into said syringe barrel,
**characterised in that** said syringe barrel is made of material permitting visual examination of contents therein; and said nozzle tip (1) has a curved neck portion (7b).

2. The syringe and nozzle tip assembly as set forth in claim 1, wherein said nozzle tip (1) includes a flange (4) and a recess (5) disposed therein and a screen (6) mounted in the recess (5) of said flange (4).

3. The syringe and nozzle tip assembly as set forth in claim 2, wherein said screen (6) and curved neck portion (7b) are integral with said nozzle tip (1) and said nozzle tip (1) and screen (6) are made of low density polyethylene.

4. The syringe and nozzle tip assembly as set forth in claim 3, wherein the openings of said screen (6) have a mesh size of about 105 microns.

## Patentansprüche

1. Ein Zusammenbau aus einer Spritze und einer Kanülenspitze, um damit eine viskose Mischung aus knochenmarkshaltigem Blut und Knochenregenerationsmaterial an einem Operationsort zu applizieren, welcher Folgendes in Kombination beinhaltet,
a) eine Spritze (3), die über einen Spritzenzylinder (2) verfügt, welcher ein vorderes Ende und eine hinteres Ende hat;
b) einen Kolben, der gleitfähig, hin und her beweglich in dem besagten Spritzenzylinder untergebracht ist;
c) einen Stempel, der an seinem vorderen Ende mit dem besagten Kolben verbunden ist, und sich rückwärtig von dem besagten Spritzenzylinder axial durch dessen besagtes hinteres Ende erstreckt; und
d) eine Kanülenspitze (1), die über einen Rand (4) und einen hülsenartigen Anteil (1a), der mit Hilfe von Reibungspassung koaxial an dem vorderen Ende des besagten Spritzenzylinders (2) angebracht ist, verfügt; wobei die besagte Kanülenspitze (1) einen Halsteil (7) hat, der über eine Passage verfügt, welche sich durch diesen hindurch erstreckt, um knochenmarkshaltiges Blut von einem Operationsort aufzusaugen und es in den besagten Spritzenzylinder zu transportieren, was dadurch charakterisiert ist, dass der besagte Spritzenzylinder aus einem Material besteht, welches eine visuelle Untersuchung der darin befindlichen Inhalte erlaubt, und dass die besagte Kanülenspitze (1) einen gebogenen Halsteil (7b) hat.

2. Der Zusammenbau aus der Spritze und der Kanülenspitze, wie es in Anspruch 1 dargelegt worden ist, worin die besagte Kanülenspitze (1) einen Rand (4) und eine Aussparung (5), welche sich in diesem befindet, enthält, sowie ein Sieb (6), das sich in der Aussparung (5) des besagten Randes (4) befindet.

3. Der Zusammenbau aus der Spritze und der Kanülenspitze, wie es in Anspruch 2 dargelegt worden ist, worin das besagte Sieb (6) und der gebogene Halsteil (7b) fest in die besagte Kanülenspitze (1) integriert sind, und die besagte Kanülenspitze (1) und das Sieb (6) aus weichem Polyethylen bestehen.

4. Der Zusammenbau aus der Spritze und der Kanülenspitze, wie es in Anspruch 3 dargelegt worden ist, worin die Öffnungen des besagten Siebs (6) eine Maschengröße von etwa 105 Mikrometern haben.

## Revendications

1. Assemblage de seringue et d'embout d'aiguille pour appliquer un mélange visqueux de sang de moelle et d'un matériau de régénération d'os à un site chirurgical, comprenant en combinaison,
a) une seringue (3) ayant un corps de seringue (2) qui a une extrémité avant et une extrémité arrière,
b) un piston monté de manière mobile de façon à pouvoir coulisser réciproquement dans ledit corps de seringue ;
c) un plongeur étant raccordé à son extrémité avant audit piston et s'étendant axialement vers l'arrière à partir dudit corps de seringue jusqu'à ladite extrémité arrière de celui-là ; et
d) un embout d'aiguille (1) ayant un rebord (4) et une partie à douille (la) qui est de manière coaxiale montée sur l'extrémité avant dudit corps de seringue (2) au moyen d'un emboîtement par frottement ; ledit embout d'aiguille (1) ayant une partie en tuyau (7) avec un passage s'étendant à travers elle pour aspirer le sang de moelle d'un site chirurgical et pour le transporter dans ledit corps de seringue,
**caractérisé en ce que** ledit corps de seringue est fait en un matériau permettant un examen visuel de ses contenus et que ledit embout d'aiguille (1) a une partie en tuyau courbée (7b).

2. Assemblage de seringue et d'embout d'aiguille comme exposé dans la revendication 1, dans lequel ledit embout d'aiguille (1) inclut un rebord (4) et un renfoncement (5) disposé dans celui-là et un écran (6) monté dans le renfoncement (5) dudit rebord (4).

3. Assemblage de seringue et d'embout d'aiguille comme exposé dans la revendication 2, dans lequel ledit écran (6) et ladite partie en tuyau courbée (7b) font partie intégrante dudit embout d'aiguille (1) et ledit embout d'aiguille (1) et ledit écran (6) sont réalisés en un polyéthylène de faible densité.

4. Assemblage de seringue et d'embout d'aiguille comme exposé dans la revendication 3, dans lequel les ouvertures dudit écran (6) ont une ouverture de maille d'environ 105 microns.
